(19) Europäisches Patentamt
European Patent Office
Office européen des brevets

(11) Numéro de publication: **0 131 522**
**B1**

(12) **FASCICULE DE BREVET EUROPÉEN**

(45) Date de publication du fascicule du brevet: **13.01.88**

(51) Int. Cl.⁴: **C 07 D 405/04,** A 01 N 47/38

(21) Numéro de dépôt: **84401458.9**

(22) Date de dépôt: **10.07.84**

(54) **Nouveaux uraciles substitués comportant en position 1 un groupement phényloxy carbonyl substitué, leur préparation et les compositions herbicides les renfermant.**

(30) Priorité: **11.07.83 FR 8311518**

(43) Date de publication de la demande: **16.01.85 Bulletin 85/3**

(45) Mention de la délivrance du brevet: **13.01.88 Bulletin 88/2**

(84) Etats contractants désignés: **BE CH DE GB IT LI NL**

(56) Documents cité: **EP-A-0 060 955** **FR-A-2 468 603**

(73) Titulaire: **ROUSSEL- UCLAF, 35, boulevard des Invalides, F-75007 Paris (FR)**

(72) Inventeur: **Tessier, Jean, 30, rue Jean Moulin, F-94300- Vincennes (FR)**
Inventeur: **Girault, Pierre, 4, rue des Abbesses, F-75018 Paris (FR)**

(74) Mandataire: **Tonnellier, Marie- José, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 131 522 B1

LIBER, STOCKHOLM 1988

**Description**

La présente invention concerne de nouveaux uraciles substitués comportant en position 1 un groupement phényloxy carbonyl substitué, leur procédé de préparation et les compositions herbicides les renfermant.

On avait déjà connaissance dans le brevet français 2 468 603 de composés uraciles de formule générale :

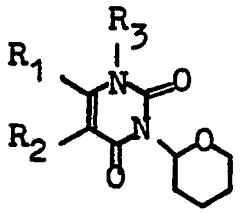

dans laquelle $R_1$ et $R_2$ représentent un radical alkyle ou forment ensemble avec les atomes de carbone auxquels ils sont liés, un homocycle carboné ou un cycle thiophénique et dans laquelle $R_3$ représente notamment un radical $CO_2Z$, Z représentant un radical alkyle, cycloalkyle, phényle ou benzyle.

Les composés de la présente invention répondent à la formule ci-dessus dans laquelle $R_1$ et $R_2$ forment ensemble un radical cyclopentyle et dans laquelle $R_3$ représente un radical $CO_2Z$, Z étant un radical phényle substitué.

Le brevet français 248603 n'envisage pas de radical $CO_2Z$ dans lequel Z représente un radical phényl substitué.

On connaissait, d'après le brevet européen EP - A - 0060955 des dérivés de la 1-phényl oxycarbonyl de la 3-(2-tétrahydropyranyl) 1, 2, 3, 4, 6, 7 hexahydro 5 H cyclopentapyrimidine, présentant des propriétés herbicides sélectives sur certaines cultures de graminés.

Il a été trouvé que la structure chimique particulière des produits de la présente invention leur conférait une activité biologique tout à fait inattendue.

Les composés de l'invention s'avèrent, en effet, doués notamment d'une activité herbicide sélective vis-à-vis du riz et peuvent être en conséquence utilisés pour le désherbage sélectif de cette culture. Au contraire, les composés du brevet français 2468603 sont dénués d'activité herbicide sélective sur le riz, comme le montrent les résultats des tests figurant ci-après.

C'est ainsi que la présente invention a pour objet les composés de formule générale (I):

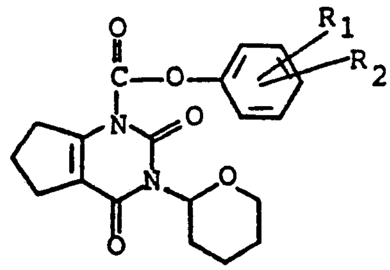

(I)

dans laquelle $R_1$ représente un radical alcoyle linéaire ou ramifié, comportant de 1 à 8 atomes de carbone, un atome d'halogène, un radical alcoxyle linéaire ou ramifié comportant de 1 à 8 atomes de carbone, un radical $-C(Hal)_3$ dans lequel Hal représente un atome de flour, de chlore ou de brome et $R_2$ représente un atome d'hydrogène.

Dans les composés de l'invention, $R_1$ représente notamment soit un radical méthyle, éthyle, propyle linéaire ou ramifié, butyle linéaire ou ramifié, pentyle linéaire ou ramifié, hexyle linéaire ou ramifié, heptyle linéaire ou ramifié, octyle linéaire ou ramifié, soit un atome de fluor, de chlore ou de brome, soit un radical méthoxyle éthoxyle, propoxyle linéaire ou ramifié, butoxyle linéaire ou ramifié, pentoxyle linéaire ou ramifié, hexyloxyle linéaire ou ramifié, heptyloxyle linéaire ou ramifié, octyloxyle linéaire ou ramifié soit un radical $-CF_3$, $-CCl_3$ ou $-CBr_3$.

Les composés de l'invention sont doués de remarquables propriétés herbicides sélectives vis-à-vis du rizé ce qui leur permet d'être utilisés pour le désherbage sélectif des cultures de riz. Ils sont également doués de propriétés herbicides sélectives vis-à-vis du coton et du maïs.

Les propriétés herbicides sélectives des composés de l'invention peuvent être mises en évidence par des tests sur des plantes représentatives des grandes familles botaniques, par exemple, par des tests sur blé, orge, maïs, folle avoine, agrostis, lolium, vulpin, panicum crusgalli, riz, betterave, chenopode, chrysanthème, gaillet, soja, coton, moutarde, amaranthe, xanthium.

La partie expérimentale exposée ci-après met en évidence l'activité herbicide sélective des composés de l'invention vis-à-vis du riz , du coton et du maïs.

L'invention a plus particulièrement pour objet les composés de formule générale (I) dans lesquels $R_2$

représente un atome d'hydrogène.

L'invention a notamment pour objet le composé répondant à la formule (I) dont le nom suit :

- la 3-(2-tétrahydropyranyl) 1,2,3,4, 6,7-hexahydro 1-(p-tolyl oxycarbonyl) 5H cyclopenta pyrimidine 2,4-dione, ainsi que les composés répondant à la formule (I), dont les noms suivent :
- la 3-(2-tétrahydropyranyl) 1,2,3,4, 6,7-hexahydro 1-(3-trifluorométhyl phénoxycarbonyl) 5H cyclopenta pyrimidine 2,4-dione ;
- la 3-(2-tétrahydropyranyl) 1,2,3,4, 6,7-hexahydro 1-(4-terbutyl phénoxycarbonyl) 5H cyclopenta pyrimidine 2,4-dione ;
- la 3-(2-tétrahydropyranyl) 1,2,3,4, 6,7-hexahydro 1-(3-méthyl phénoxy carbonyl) 5H cyclopenta pyrimidine 2,4-dione ;
- la 3-(2-tétrahydropyranyl) 1,2,3,4, 6,7-hexahydro 1-(2-méthyl phénoxycarbonyl) 5H cyclopenta pyrimidine 2,4-dione ;
- la 3-(2-tétrahydropyranyl) 1,2,3,4, 6,7-hexahydro 1-(α-naphtyloxycarbonyl) 5H cyclopenta pyrimidine 2,4-dione ;
- la 3-(2-tétrahydropyranyl) 1,2,3,4, 6,7-hexahydro 1-(4-chlorophénoxycarbonyl) 5H cyclopenta pyrimidine 2,4-dione ;
- la 3-(2-tétrahydropyranyl) 1,2,3,4, 6,7-hexahydro 1-(4-méthoxy phénoxy carbonyl) 5H cyclopenta pyrimidine 2,4-dione.

L'invention a également pour objet un procédé de préparation des composés de formule générale (I), caractérisé en ce que l'on fait réagir sur la 3-(2-tétrahydropyranyl) 1,2,3, 4,6,7-hexahydro 5H cyclopenta pyrimidine 2,4-dione, préparée selon le brevet français 2029250, une base forte, puis un composé de formule générale :

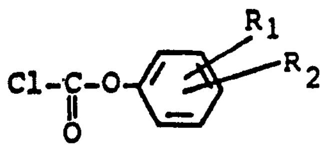

(III)

dans laquelle $R_1$ et $R_2$ conservent les significations données ci-dessus pour obtenir le composé de formule (I) correspondant.

Dans un mode d'exécution préféré de l'invention, la base forte est l'hydrure de sodium.

L'invention a aussi pour objet les compositions pesticides notamment herbicides caractérisées en ce qu'elles contiennent comme matière active au moins l'un des composés tels que definis précédemment.

L'invention a notamment pour objet les compositions herbicides utilisées pour la culture du riz, du coton ou du maïs, caractérisées en ce qu'elles contiennent comme matière active au moins l'un des composés tels que définis précédemment.

L'invention a aussi pour objet l'application des compositions telles que définies précédemment au désherbage sélectif des cultures de riz, de maïs et de coton.

Les compositions selon l'invention peuvent se présenter sous forme de poudres, granulés, suspensions, émulsions, solutions contenant le principe actif, par exemple, en mélange avec un véhicule et/ou un agent tensio-actif anionique, cationique ou non ionique assurant entre autres, une dispersion uniforme des substances de la composition. Le véhicule utilisé peut être un liquide, tel que l'eau, l'alcool, les hydrocarbures ou autres solvants organiques, une huile minérale, animale ou végétale, ou une poudre, telle que le talc, les argiles, les silicates, le kieselguhr.

Les compositions solides, présentées sous forme de poudre pour poudrage, de poudres mouillables ou de granulés, peuvent être préparées par broyage du composé actif avec un solide inerte ou par imprégnation d'un support solide avec une solution du principe actif dans un solvant que l'on évapore ensuite.

Outre un véhicule et/ou un agent tensio-actif, ces compositions contiennent, comme principe actif, un ou plusieurs composés de formule (I), ainsi qu'éventuellement d'autres pesticides et des substances présentant des propriétés influant sur la croissance des plantes.

Les compositions de l'invention sont, bien entendu, appliquées à des doses suffisantes pour exercer leurs activités herbicides. Les doses de matière active dans les compositions varient notamment en fonction des végétaux à détruire, de la nature du terrain, des conditions atmosphériques et de l'état d'avancement de la végétation à détruire.

Les compositions herbicides selon l'invention contiennent en général de 10 à 80 % en poids et, de préférence, de 10 à 50 % en poids de matière active.

Lors de l'utilisation des composés I comme herbicide, les quantités de substance active appliquées varient en prélevée de préférence, entre 0,1 et 5 Kg/ha et en post-levée, de préférence entre 0,1 et 800 g/ha.

Les exemples suivants illustrent l'invention sans toutefois la limiter.

**EXEMPLE 1:**

3(2-tétrahydropyranyl) 1,2,2,4,6,7-hexahydro 1-(p-tolyl oxycarbonyl) 5H cyclopenta pyrimidine 2,4-dione.

Dans 130 $cm^3$ de tétrahydrofuranne anhydre et 13 $cm^3$-d'hexaméthyl phosphotriamide, on introduit 15 g de 3(2-tétrahydropyranyl) 1,2,3,4,6,7-hexahydro 5H cyclopenta pyrimidine 2,4-dione préparé selon le brevet français 2029250, agite pendant 20 minutes à 20°C, introduit en plusieurs fois, à 0°C, 3,35 g de suspension d'hydrure de sodium à 50 % dans l'huile, agite pendant 1 heure et 45 minutes, introduit goutte à goutte, 13 g de chloroformiate de p-tolyle préparé à partir du p-crésol, agite à 20°C pendant 17 heures, verse dans l'eau glacée, ajoute de l'éther isopropylique, isole par essorage l'insoluble formé, le sèche et obtient 15,5 g de produit recherché F=136°C.

**EXEMPLE 2:**

(2-tétrahydropyranyl) 1,2,3,4,6,7-hexahydro 1-(3-trifluorométhyl phénoxy carbonyl) 5H cyclopenta pyrimidine 2,4-dione.

De manière analogue à celle de l'exemple 1, en utilisant le chloroformiate de 3-trifluorométhyl phényle préparé à partir du 3-trifluorométhylphénol, on obtient le produit recherché F=130°C.

**EXEMPLE 3:**

3(2-tétrahydropyranyl) 1,2,3,4,6,7-hexahydro 1-(4-terbutyl phénoxy carbonyl) 5H cyclopenta pyrimidine 2,4-dione.

De manière analogue à celle de l'exemple 1, en utilisant le chloroformiate de 4-terbutyl phényle préparé à partir du 4-terbutylphénol, on obtient le produit recherché F=135°C.

**EXEMPLE 4:**

3(2-tétrahydropyranyl) 1,2,3,4,6,7-hexahydro 1-(3-méthyl phénoxy carbonyl) 5H cyclopenta pyrimidine 2,4-dione.

De manière analogue à celle de l'exemple 1, en utilisant le chloroformiate de m-tolyle préparé à partir du m-crésol, on obtient le produit recherché F=155°C.

**EXEMPLE 5:**

3(2-tétrahydropyranyl) 1,2,3,4,6,7-hexahydro 1-(2-méthyl phénoxy carbonyl) 5H cyclopenta pyrimidine 2,4-dione.

De manière analogue à celle de l'exemple 1, en utilisant le chloroformiate d'O-tolyle préparé à partir de 1'O-crésol, on obtient le produit recherché.

Spectre de RMN (deutérochloroforme)

pics de 1,5 à 3,16 ppm des hydrogènes de

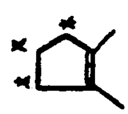

pics de 1,5 à 3,16 ppm des hydrogènes de

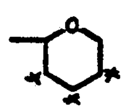

pic à 2,31 ppm des hydrogènes du méthyle de l'ortho tolyle.

pics de 3,3 à 4,3 ppm de l'hydrogène de

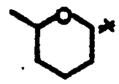

pics à 5,85-5,88 et 6,03-6,07 ppm de l'hydrogène de

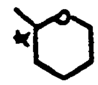

pic à 7,23 ppm des hydrogènes du noyau aromatique.

**EXEMPLE 6:**

3(2-tétrahydropyranyl) 1,2,3,4,6,7-hexahydro 1-(α-naphtyloxy carbonyl) 5H cyclopenta pyrimidine 2,4-dione.

De manière analogue à celle de l'exemple 1, en utilisant le chloroformiate d'α-naphtyle préparé par exemple selon Am. Soc. 47.2609 (1925), on obtient le produit recherché F=160° C.

**EXEMPLE 7:**

3(2-tétrahydropyranyl) 1,2,3,4,6,7-hexahydro 1-(4-chlorophénoxy carbonyl) 5H cyclopenta pyrimidine 2,4-dione,

De manière analogue à celle de l'exemple 1, en utilisant le chloroformiate de 4-chloro phényle préparé par exemple selon Am. Soc. 47-2609 (1925), on obtient le produit recherché F=126° C.

**EXEMPLE 8:**

3(2-tétrahydropyranyl) 1,2,3,4,6,7-hexahydro 1-(4-méthoxy phénoxy carbonyl) 5H cyclopenta pyrimidine 2,4-dione.

De manière analogue à celle de l'exemple 1, en utilisant le chloroformiate de 4-méthoxy phényle préparé à partir du 4-méthoxyphénol, on obtient le produit recherché F=110° C.

**EXEMPLE 9:**

concentré émulsifiable selon l'invention.

On a préparé une composition contenant en poids 15 % de produit de l'exemple 1, 6,4 % d'atlox 4851 (triglycéride oxy éthylène combiné avec un sulfonate, indice d'acide 1,5) 3,2 % d'atlox 4855 (triglycéride oxyéthylène combiné avec un sulfonate, indice d'acide 3) et 75,4 % de xylène.

**EXEMPLE 10:**

poudre mouillable selon l'invention.

On a préparé une poudre mouillable contenant 25 % de composé de l'exemple 4, 15 % d'ekapersol (produit de condensation du naphtalène sulfonate de sodium, 0,5 % de brecolane NVA (alcoyl naphtalène sulfonate de sodium) 34,5 % de zéozil 39 (silice hydratée synthétique obtenue par précipitation) et 25 % de vercoryl "S" (kaolin colloïdal).

**EXEMPLE 11**

Etude de l'activité herbicide de pré-émergence des composés de l'invention.

Les végétaux utilisés (triticum sativum, hordeum sp., zea mays, avena ludoviciana, agrostis tenuis, lolium perenne, alopecurus myosuroides, panicum crusgalli, oryza sativa, beta vulgaris, chenopodium album, chrysanthemum coronarium, galium aparine, glycine hispida, gossypium spp., brassica sp., amarantus retroflexus, xanthium spinosum) sont cultivés en bac de culture ( 23 X 14 X 4 cm), à double fond, l'arrosage s'effectuant par dessous. Les espèces sont placées, a raison de 20 graines par espèce, en lignes espacées de 3 cm, dans un bac unique, et il y a 4 essais pour chaque concentration. Les conditions de culture sont les suivantes : température : 20°C ± 2°C, humidité : 60 % environ, éclairement : par tube fluorescent (lumière du jour + blanc brillant) de six heures à vingt-deux heures, chaque jour. Le mélange terreux utilisé est composé de 10 volumes de terre franche, de 10 volumes de sable de rivière et de 2 volumes de tourbe. On effectue un essai de pré-émergence. Le traitement est effectué vingt-quatre heures après le semis et le premier arrosage est effectué par aspersion de façon à entraîner une partie du produit au niveau des graines.

Le produit à étudie est appliqué, dans des conditions standard à l'aide d'un micro-pulvérisateur, à des doses correspondant à 2,5 - 1,25, et 0,625 Kg/ha et une dilution correspondant à 560 l/ha.

On effectue un essai avec un témoin non traité. Cet essai comporte le même nombre de plantules que les plantules traitées.

Le contrôle final est effectué par dénombrement de plantules vingt et un jours après traitement.

Les résultats sont exprimés en pourcentage de réduction du nombre de plants (pourcentage de mortalité) M:

$$M = \frac{\text{(Nombre de plantules témoin)-(nombre de plantules traitées toujours vivantes)}}{\text{Nombre de plantules témoin}} \times 100$$

Les résultats expérimentaux obtenus avec le composé de l'exemple 1 sont résumés dans le tableau suivant:

Pourcentage de mortalité

| Plantes traitées | Dose en Kg/ha 2,5 | 1,25 | 0,625 |
|---|---|---|---|
| Triticum sativum | 100 | 17 | 50 |
| Hordeum sp. | 100 | 100 | 100 |
| Zea mays | 0 | 0 | 0 |
| Avena ludoviciana | 100 | 63 | 25 |
| Agrostis tenuis | 100 | 100 | 100 |
| Lolium perenne | 90 | 80 | 63 |
| Alopecurus myosuroides | 100 | 100 | 100 |
| Panicum crusgalli | 50 | 32 | 14 |
| Oryza sativa | 0 | 0 | 0 |
| Beta vulgaris | 100 | 86 | 43 |
| Chenopodium album | 100 | 100 | 100 |
| Chrysanthemum coronarium | 100 | 79 | 39 |
| Galium aparine | 100 | 100 | 85 |
| Glycine hispida | 100 | 92 | 42 |
| Gossypium spp. | 0 | 0 | 0 |
| Brassica sp. | 100 | 100 | 100 |
| Amarantus retroflexus | 61 | 55 | 18 |
| Xanthium spinosum | 100 | 67 | 33 |

Conclusion: Ces résultats expérimentaux montrent la bonne sélectivité du composé de l'exemple 1 sur riz, sur coton et aussi sur maïs.

**EXEMPLE 12**

Etude comparative de l'activité herbicide sur riz en préemergence du composé de l'exemple 1(dénommé composé A) et de la 1-(méthoxycarbonyl) 3-(2-tétrahydropyranyl) 5-éthyl 6-méthyl 1,2,3,4-tétrahydropyrimidine 2,4-dione (produit de l'exemple 4 du brevet français 2468603(dénommé ci-après composé B).

En répétant le test de l'exemple 11 sur oryza sativa, avec les composés A et B, on a trouvé les résultats suivants:

Pourcentage de mortalité

Plante traitée dose en Kg/ha

Oriza Sativa>k 2,5>k>k 1,5>k>k 0,625>k>k Composé A>k 0>k>k 0>k>k 0>k>k Composé B>k 100>k>k 100>k>k 100>k>k>t

Conclusion: Le composé A présente une sélectivité totale sur Oriza Sativa, alors que le composé B détruit complètement la culture.

**Revendications**

1. Les composés de formule générale (I):

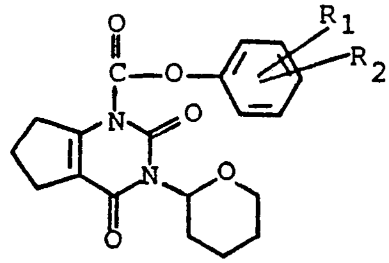

(I)

dans laquelle $R_1$ représente un radical alcoyle linéaire ou ramifié, comportant de 1 à 8 atomes de carbone, un atome d'halogène, un radical alcoxyle linéaire ou ramifié comportant de 1 à 8 atomes de carbone, un radical $-C(Hal)_3$ dans lequel Hal représente un atome de fluor, de chlore ou de brome et $R_2$ représente un atome d'hydrogène.

2. Les composés de formule générale (I), selon la revendication 1, dans lesquels $R_2$ représente un atome d'hydrogène.

3. Le composé répondant à la formule (I) telle que définie à la revendication 1 dont le nom suit:
- la 3-(2-tétrahydropyranyl) 1,2,3,4 6,7-hexahydro 1-(p-tolyl oxycarbonyl) 5H cyclopenta pyrimidine 2,4-dione, ainsi

4. Les composés répondant à la formule (I) telle que définie à la revendication 1 dont les noms suivent:
- la 3-(2-tétrahydropyranyl) 1,2,3,4, 6,7-hexahydro 1-(3-trifluorométhyl phénoxycarbonyl) 5H cyclopenta pyrimidine 2,4-dione ;
- la 3-(2-tétrahydropyranyl) 1,2,3,4, 6,7-hexahydro 7-(4-terbutyl phénoxycarbonyl) 5H cyclopenta pyrimidine 2,4-dione ;
- la 3-(2-tétrahydropyranyl) 1,2,3,4, 6,7-hexahydro 1-(3-méthyl phénoxy carbonyl) 5H cyclopenta pyrimidine 2,4-dione ;
- la 3-(2-tétrahydropyranyl) 1,2,3,4, 6,7-hexahydro 1-(2-méthyl phénoxycarbonyl) 5H cyclopenta pyrimidine 2,4-dione ;
- la 3-(2-tétrahydropyranyl) 1,2,3,4, 6,7-hexahydro 1-(α-naphtyloxycarbonyl) 5H cyclopenta pyrimidine 2,4-dione ;
- la 3-(2-tétrahydropyranyl) 1,2,3,4, 6,7-hexahydro 1-(4-chlorophénoxycarbonyl) 5H cyclopenta pyrimidine 2,4-dione ;
- la 3-(2-tétrahydropyranyl) 1,2,3,4, 6,7-hexahydro 1-(4-méthoxy phénoxy carbonyl) 5H cyclopenta pyrimidine 2,4-dione.

5. Procédé de préparation des composés de formule générale (I) tels que définis à l'une quelconque des revendications 1 à 4, caractérisé en ce que l'on fait réagir sur la 3-(2-tétrahydropyranyl) 1,2,3,4,6,7-hexahydro 5H cyclopenta pyrimidine 2,4-dione, une base forte, puis un composé de formule générale:

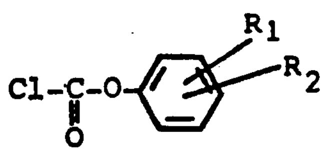

**(III)**

dans laquelle $R_1$ et $R_2$ conservent les significations de la revendication 1 pour obtenir le composé de formule (I) correspondant.

6. Procédé selon la revendication 5, caractérisé en ce que la base forte est l'hydrure de sodium.

7. Les compositions herbicides caractérisées en ce qu'elles contiennent comme matière active au moins l'un des composés selon l'une quelconque des revendications 1 à 4.

8. Les compositions herbicides selon la revendication 7 utilisées pour la culture du riz, caractérisées en ce qu'elles contiennent comme matière active au moins l'un des composés selon l'une quelconque des revendications 1 à 4.

9. Les compositions herbicides selon la revendication 7 utilisées pour la culture du coton et du maïs caractérisées en ce qu'elles contiennent comme matière active au moins l'un des composés selon l'une quelconque des revendications 1 à 4.

10. Application des compositions selon la revendication 7 au désherbage sélectif des cultures de riz.

11. Application des compositions selon la revendication 7 au désherbage sélectif des cultures de coton et de maïs.

**Patentansprüche**

1. Verbindungen der allgemeinen Formel (I)

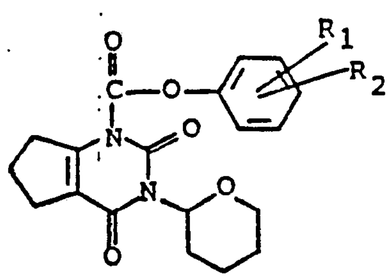

(I)

worin $R_1$ einen linearen oder verzweigten Alkylrest mit 1 bis 8 Kohlenstoffatomen, ein Halogenatom, einen linearen oder verzweigten Alkoxyrest mit 1 bis 8 Kohlenstoffatomen oder einen Rest $-C(Hal)_3$, worin Hal ein Fluor-, Chlor- oder Bromatatom darstellt, bedeutet, und $R_1$ für Wasserstoff steht.

2. Verbindungen der allgemeinen Formel (I) gemäß Anspruch 1, worin $R_2$ ein Wasserstoffatom bedeutet.

3. Verbindung der Formel (I) gemäß Anspruch 1, mit der folgenden Bezeichnung:
- 3-(2-Tetrahydropyranyl)-1,2,3,4,6,7-hexahydro-1-(p-tolyl-oxycarbonyl)-5H-cyclopenta-pyrimidin-2,4-dion.

4. Verbindungen der Formel (I) gemäß Anspruch 1 mit den folgenden Bezeichnungen:
- 3-(2-Tetrahydropyranyl)-1,2,3,4,6,7-hexahydro-1-(3-trifluor-methyl-phenoxycarbonyl)-5H-cyclopenta-pyrimidin-2,4-dion;
- 3-(2-Tetrahydropyranyl)-1,2,3,4,6,7-hexahydro-1-(4-tert.-butyl-phenoxycarbonyl)-5H-cyclopenta-pyrimidin-2,4-dion;
- 3-(2-Tetrahydropyranyl)-1,2,3,4,6,7-hexahydro-1-(3-methyl-phenoxycarbonyl)-5H-cyclopenta-pyrimidin-2,4-dion;
- 3-(2-Tetrahydropyranyl)-1,2,3,4,6,7-hexahydro-1-(2-methyl-phenoxycarbonyl)-5H-cyclopenta-pyrimidin-2,4-dion;
- 3-(2-Tetrahydropyranyl)-1,2,3,4,6,7-hexahydro-1-(α-naphthyl-oxycarbonyl)-5H-cyclopenta-pyrimidin-2,4-dion;
- 3-(2-Tetrahydropyranyl)-1,2,3,4,6,7-hexahydro-1-(4-chlorophenoxycarbonyl)-5H-cyclopenta-pyrimidin-2,4-dion;,
- 3-(2-Tetrahydropyranyl)-1,2,3,4,6,7-hexahydro-1-(4-methoxy-phenoxycarbonyl)-5H-cyclopenta-pyrimidin-2,4-dion.

5. Verfahren zur Herstellung der Verbindungen der allgemeinen Formel (I) gemäß einem der Ansprüche 1 bis 4, dadurch gekennzeichnet, daß man mit 3-(2-Tetrahydropyranyl)-1,2,3,4,6,7-hexahydro-5H-cyclopenta-pyrimidin-2,4-dion eine starke Base, danach eine Verbindung der allgemeinen Formel

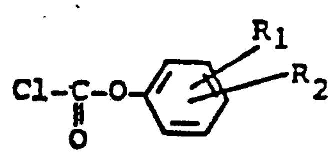

(III)

worin $R_1$ und $R_2$ die in Anspruch 1 angegebene Bedeutung besitzen, umsetzt, um die entsprechende Verbindung der Formel (I) zu erhalten.

6. Verfahren gemäß Anspruch 5, dadurch gekennzeichnet, daß die starke Base Natriumhydrid ist.

7. Herbizide Zusammensetzungen, dadurch gekennzeichnet, daß sie als Wirkstoff zumindest eine der Verbindungen gemäß einem der Ansprüche 1 bis 4 enthalten.

8. Herbizide Zusammensetzungen gemäß Anspruch 7 für die Verwendung bei Reiskulturen, dadurch gekennzeichnet, daß sie als Wirkstoff zumindest eine der Verbindungen gemäß einem der Ansprüche 1 bis 4 enthalten.

9. Herbizide Zusammensetzungen gemäß Anspruch 7 für die Verwendung bei Baumwolle- und Maiskulturen, dadurch gekennzeichnet, daß sie als Wirkstoff zumindest eine der Verbindungen gemäß einem der Ansprüche 1 bis 4 enthalten.

10. Verwendung der Zusammensetzungen gemäß Anspruch 7 für die selektive Unkrautbekämpfung bei Reiskulturen.

11. Verwendung der Zusammensetzung gemäß Anspruch 7 für die selektive Unkrautbekämpfung bei Baumwolle- und Maiskulturen.

**Claims**

1. Compounds with the general formula (I):

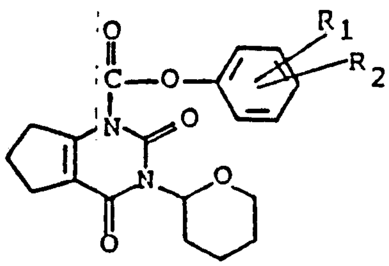

(I)

in which $R_1$ represents a linear or branched alkyl radical, containing from 1 to 8 carbon atoms, a halogen atom, a linear or branched alkoxyl radical containing from 1 to 8 carbon atoms, a $-C(Hal)_3$ radical in which Hal represents a fluorine, chlorine or bromine atom and $R_2$ represents a hydrogen atom.

2. Compounds with the general formula (I), according to claim 1, in which $R_2$ represents a hydrogen atom.

3. The compound answering to the general formula (I) as defined in claim 1 of which the name follows:
3-(2-tetrahydropyranyl)-1,2,3,4,6,7-hexahydro-1-(p-tolyloxycarbonyl)-5H-cyclopentapyrimidine-2,4-dione.

4. Compounds answering to the formula (I) as defined in claim 1 of which the names follow:
3-(2-tetrahydropyranyl)-1,2,3,4,6,7-hexahydro-1-(3-trifluoromethyl-phenoxycarbonyl)-5H-cyclopentapyrimidine-2,4-dione;
3-(2-tetrahydropyranyl)-1,2,3,4,6,7-hexahydro-1-(4-tertbutylphenoxy-carbonyl)-5H-cyclopentapyrimidine-2,4-dione;
3-(2-tetrahydropyranyl)-1,2,3,4,6,7-hexahydro-1-(3-methylphenoxycarbonyl) -5H-cyclopentapyrimidine-2,4-dione;
3-(2-tetrahydropyranyl)-1,2,3,4,6,7-hexahydro-1-(2-methylpnenoxycarbonyl) -5H-cyclopentapyrimidine-2,4-dione;
3-(2-tetrahydropyranyl)-1,2,3,4,6,7-hexahydro-1-(alpha-naphthyloxy-carbonyl)-5H-cyclopentapyrimidine-2,4-dione;,
3-(2-tetrahydropyranyl)-1,2,3,4,6,7-hexahydro-1-(4-chlorophenoxycarbonyl) -5H-cyclopentapyrimidine-2,4-dione;
3-(2-tetrahydropyranyl)-1,2,3,4,6,7-hexahydro-1-(4-methoxyphenoxy-carbonyl)-5H-cyclopentapyrimidine-2,4-dione.

5. Preparation process for compounds with the general formula (I) as defined in any one of the claims 1 to 4, characterized in that a strong base, and then a compound with the general formula:

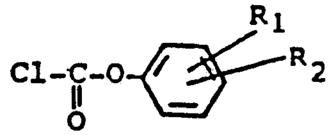

(III)

in which $R_1$ and $R_2$ keep the significances of claim 1, are made to react on 3-(2-tetrahydropyranyl)-1,2,3,4,6,7-hexahydro-5H-cyclopentapyrimidine-2,4-dione, in order to obtain the corresponding compound with the formula (I).

6. Process according to claim 5, characterized in that the strong base is sodium hydride.

7. Herbicide compositions characterized in that they contain as active material at least one of the compounds

according to any one of the claims 1 to 4.

8. Herbicide compositions according to claim 7 used for rice cultivation characterized in that they contain as active material at least one of the compounds according to any one of the claims 1 to 4.

9. Herbicide compositions according to claim 7 used for cotton and corn cultivation characterized in that they contain as active material at least one of the compounds according to any one of the claims 1 to 4.

10. Application of the compositions according to claim 7 for selective weeding of rice cultivations.

11. Application of the compositions according to claim 7 for selective weeding of cotton and corn cultivations.